# EUROPEAN PATENT APPLICATION

(11) **EP 4 659 754 A1**
(43) Date of publication of application: **10.12.2025**
(21) Application number: 23919247.9
(22) Date of filing: 05.06.2023
(51) Int. Cl.: A61K 33/00, C12N 5/0783, A61K 39/395, A61K 35/17, A61P 35/00, A61P 35/02

(54) **USE OF LITHIUM-CONTAINING COMPOUNDS IN TREATMENT OF TUMORS**

(30) Priority: 30.01.2023 CN 202310117114
(71) Applicant: Institute of Basic Medical Sciences Chinese Academy of Medical Sciences, Beijing 100005 (CN)
(72) Inventor: HUANG, Bo, Beijing 100005 (CN)
(74) Representative: Cabinet Beau de Loménie
(86) International application number: PCT/CN2023/098271
(87) International publication number: WO 2024/159680

(57) **Abstract**

Disclosed is the use of a lithium-containing compound in the treatment of a tumor. Specifically, the present invention relates to use of a lithium-containing compound in preparing a medicament for treating a tumor.

## Description

### FIELD OF THE INVENTION

The present invention relates to the technical field of tumor treatment. Specifically, the present invention relates to use of a lithium-containing compound in preparing a medicament for treating a tumor.

### BACKGROUND OF THE INVENTION

Cancer is an important global public health problem, and its incidence and death toll are on an increasing trend year by year. It has become an urgent need for social development to provide optimal treatment methods for patients with malignant tumors. At present, clinical anti-tumor treatment is still dominated by traditional surgery, radiotherapy and chemotherapy. Different treatment regimens have different treatment effects, and may cause varying amounts of damage or side effects to the body. The "tumor immunotherapy" that has emerged in recent years has shown unprecedented clinical efficacy. Compared with traditional tumor treatment methods, immunotherapy has stronger targeting and lower toxicity and side effects, but only a minority of patients are clinically benefited. Due to the persistent high incidence of cancer, the demand for anti-tumor products is increasing, and there is also an urgent need for transformation of China's original and internationally leading achievements in tumor treatment technologies. For patients with tumor, the activation and maintenance of tumor-specific CD8⁺ T cells is the key to the success of anti-tumor immunity. However, in the tumor microenvironment, there are a large number of immunosuppressive cell subsets such as regulatory T cells (Tregs), tumor-associated macrophages (TAMs), myeloid-derived suppressor cells (MDSCs), and inhibitory molecules such as PD-1, CTLA-4, IL-10, TGF-β, as well as immunosuppressive metabolites such as lactic acid and kynurenine, which jointly inhibit the recognition, activation, proliferation and effect of anti-tumor CD8⁺ T cells, thereby reducing or even eliminating the effect of immunotherapies.

Metabolism is closely related to important biological events such as survival, proliferation and function of cells. The Warburg effect is a common metabolic characteristic shared by tumor cells and even activated immune cells. That is, tumor cells still take glycolysis as the main metabolic mode under aerobic conditions, thereby producing a large amount of metabolic by-product, lactic acid, which can reach a concentration of 40 mM or even higher in the tumor microenvironment. Studies have demonstrated that lactic acid in the tumor microenvironment plays a role in promoting tumorigenesis and tumor development by promoting the function of Tregs, antigen presentation of DCs, up-regulating PD1 expression of T cells and polarization of M2 tumor-associated macrophages, and inhibiting the function of anti-tumor CD8⁺ T cells, etc. In patients with malignant tumor, a higher level of lactic acid is strongly associated with a decrease in the number of tumor tissue infiltrating CD8⁺ T cells and a poor prognosis. Therefore, lactic acid in the tumor microenvironment is a key immune checkpoint molecule and a potential target for a new generation of tumor immunotherapy.

It is worth noting that despite its immunosuppressive effects, lactic acid is a physiologically important energy molecule that is used for gluconeogenesis via the Cori cycle or direct oxidation. It is one of the most preferred forms of energy used by the body, even more preferred than glucose. This suggests that lactic acid in the microenvironment can provide energy to immune cells, thereby enhancing their anti-tumor function. In other words, promoting the uptake and utilization of lactic acid by T cells may be a potential anti-tumor immunotherapy regimen. Therefore, we have found a clinical drug with established pharmacological and toxicological effects, which can not only reduce the inhibition of lactic acid on tumor-responsive CD8⁺ T cells, but also enhance the anti-tumor effect of tumor-specific T cells by mobilizing the oxidation of lactic acid in mitochondria and thus reducing the inhibition of lactic acid on tumor-specific T cells and providing them with energy.

### SUMMARY OF THE INVENTION

In a first respect, the present invention provides use of a lithium-containing compound in preparing a medicament for treating a tumor. Specifically, the lithium-containing compound of the present invention play a role in treating tumor by promoting the utilization of lactic acid in the microenvironment by tumor-specific CD8⁺ T cells and enhancing the anti-tumor function of CD8⁺ T cells.

In particular embodiments, the lithium-containing compound of the present invention is selected from the group consisting of lithium carbonate, lithium chloride, lithium hydroxide, n-butyl lithium, lithium sulfate, lithium citrate and lithium orotate.

In another aspect, the present invention provides use of a lithium-containing compound in enhancing T cell-based tumor immunotherapy.

Specifically, the present invention provides use of a lithium-containing compound in preparing a medicament for enhancing immune checkpoint inhibitor and/or adoptive T cell therapy.

An immune checkpoint inhibitor can exert anti-tumor effect by blocking immunosuppressive signals in the tumor microenvironment and restoring the function of immune cells.

As used herein, the immune checkpoint inhibitor includes, but is not limited to, molecules that inhibit the function of immune checkpoint molecule groups such as CTLA-4, PD-1, or PD-L1 as a ligand thereof.

Specifically, the present invention provides use of a lithium-containing compound in preparing a medicament for enhancing the anti-tumor activity of a PD-1 antibody.

In one embodiment, the lithium-containing compound is selected from the group consisting of lithium carbonate, lithium chloride, lithium hydroxide, n-butyl lithium, lithium sulfate, lithium citrate and lithium orotate, as well as other lithium-containing compounds that can be used or have been used in medicine.

In one embodiment, the lithium-containing compound is formulated into a dosage form for oral, intradermal, subcutaneous, intraperitoneal or intravenous administration.

In one embodiment, the PD-1 antibody includes, but is not limited to, nivolumab and pembrolizumab.

In another aspect, the present invention provides an anti-tumor pharmaceutical composition comprising a lithium-containing compound and a PD-1 antibody, optionally, the lithium-containing compound and the PD-1 antibody may exist in different preparations separately.

In further embodiments, the lithium-containing compound of the present invention can be used to treat any solid cancer as well as blood cancer. For example, mention may be made of head and neck cancer, esophageal cancer, gastric cancer, colon cancer, rectal cancer, liver cancer, gallbladder cancer, cholangiocarcinoma, pancreatic cancer, lung cancer, breast cancer, ovarian cancer, bladder cancer, prostate cancer, testicular cancer, bone and soft tissue sarcoma, malignant lymphoma, leukemia, cervical cancer, skin cancer, brain tumor, melanoma, etc.

In another aspect, the present invention provides a method for treating a tumor, including administering a therapeutically effective amount of a lithium-containing compound to a subject in need thereof, preferably further administering a T cell-based tumor immunotherapy to the subject, more preferably, the T cell-based tumor immunotherapy is selected from immune checkpoint inhibitor and/or adoptive T cell therapy, most preferably, the immune checkpoint inhibitor and/or adoptive T cell therapy is administered simultaneously, sequentially or separately with the lithium-containing compound.

As used herein, the term "treat", "treating" or "treatment" includes treating a disease or condition described herein in a subject such as a human, and includes: (i) suppressing the disease or condition, i.e., preventing it from occurring; (ii) alleviating the disease or condition, i.e., causing the condition to regress; (iii) slowing the progression of the disease; and/or (iv) suppressing, alleviating, or slowing the progression of one or more of the symptoms of the disease or condition.

The terms "patient", "subject", "individual", etc., are used interchangeably herein and, whether *in vitro* or *in situ,* refer to any animal or cells thereof that can be adapted to the methods described herein. In some non-restricting embodiments, the patient, subject or individual is a human.

The specific embodiments below are provided in the present specification in order to illustrate the present invention in more details, and are illustrated with reference to the attached drawings, but the solutions of the present disclosure are not limited thereto.

### DESCRIPTION OF THE DRAWINGS

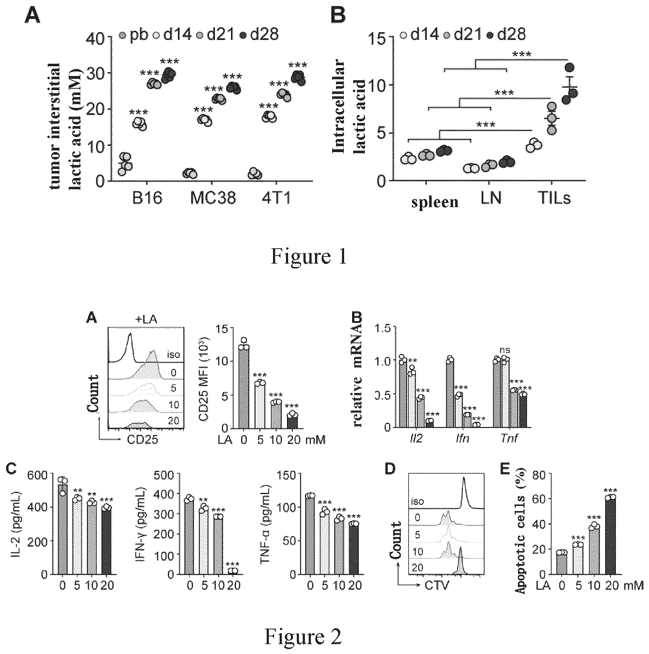

Figure 1 shows a gradual increase in lactic acid levels on Days 14, 21 and 28 after tumor inoculation. A of Figure 1 shows lactic acid levels in tumor interstitial fluid. B of Figure 1 shows intracellular lactic acid levels, with a gradual increase in intracellular lactic acid levels in tumor-infiltrating lymphocytes compared with spleen and lymph nodes.
Figure 2 shows that lactic acid significantly inhibits the activation and proliferation of CD8⁺ T cells in a dose-dependent manner, and significantly promotes the apoptosis of CD8⁺ T cells. A of Figure 2 shows that LA down-regulates CD25 in a dose-dependent manner. B of Figure 2 shows that LA down-regulates the mRNA expression of effector molecules IL-2, IFN-γ and TNF-α in a dose-dependent manner. C of Figure 2 shows that LA down-regulates the protein expression levels of the effector molecules IL-2, IFN-γ and TNF-α in a dose-dependent manner. D of Figure 2 shows that LA inhibits the proliferation of CD8⁺ T cells in a dose-dependent manner in CTV proliferation detection experiments. E of Figure 2 shows that LA promotes the apoptosis of CD8⁺ T cells in a dose-dependent manner.

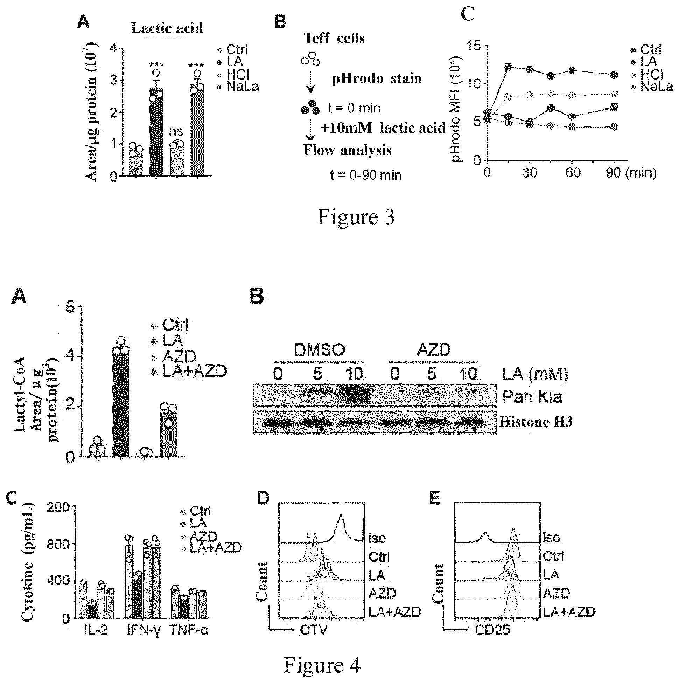

Figure 3 shows the entry of lactate and hydrogen ions into cells. A of Figure 3 shows the intracellular lactate contents after treating with lactic acid/lactate/hydrochloric acid for 30 min, as detected by LC-MS/MS, and it is found that lactic acid can quickly enter CD8⁺ effector T cells. B of Figure 3 shows the protocol for labeling CD8⁺ T cells with pHrodo acidic probes. C of Figure 3 shows that lactic acid acidifies the cytoplasm more rapidly, as detected by flow cytometry. The above results show that lactic acid (comprising a lactate and a hydrogen ion) can quickly enter CD8⁺ T cells.
Figure 4 shows that entrance of exogenous lactic acid into the nucleus results in lactylation modification of histones, thereby regulating the function of CD8⁺ T cells, and this modification can be blocked by AZD, an inhibitor of the lactate transporter MCT1. A-E of Figure 4 show the detection of lactyl-CoA contents by high performance liquid chromatography-mass spectrometry (LC-MS/MS) after treating CD8⁺ T cells with exogenous lactic acid (A of Figure 4), the detection of histone lactylation levels by Western blotting (B of Figure 4), the expression and secretion of the effectors IL-2, IFN-γ and TNF-α of CD8⁺ T cells after treating with lactate (C of Figure 4), CTV proliferation (D of Figure 4), and CD25 expression (E of Figure 4), respectively.

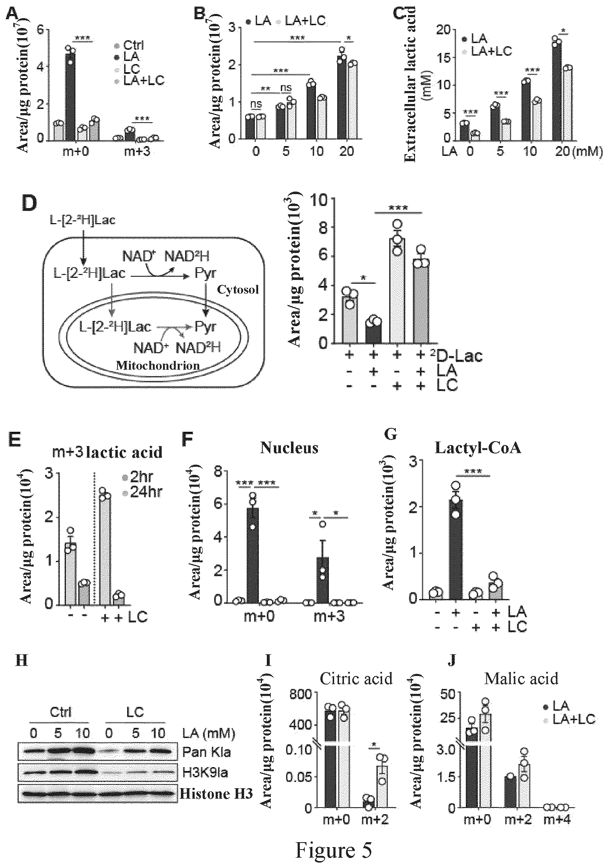

Figure 5 shows that LC introduces exogenous lactic acid into mitochondria for oxidative metabolism. A of Figure 5 shows the detection of intracellular lactic acid contents by LC-MS/MS after co-treating CD8⁺ T cells with LA and LC *in vitro* for 24 hours, and it is found that LC significantly down-regulates the intracellular lactic acid contents. B of Figure 5 shows the changes in intracellular lactic acid contents in LC-treated and control CD8⁺ T cell groups after treating with LA at different concentrations for 30 minutes. C of Figure 5 shows the changes in extracellular lactic acid. D of Figure 5 shows that lactic acid enters the mitochondria through direct transport, as found by [2-²H]-lactic acid labeling experiments. E of Figure 5 shows a rapid decrease in lactic acid in CD8⁺ T cells treated with LC, as found by M+3 ¹³C lactic acid labeling experiments. F of Figure 5 shows the changes in lactic acid content in the nucleus after co-treating with LA and LC. G of Figure 5 shows the changes in intracellular lactyl-CoA contents after co-treating with LA and LC. H of Figure 5 shows the changes in histone lactylation modification in cells co-treated with LA and LC. I of Figure 5 shows the M+0/M+2 citric acid content after ¹³C lactic acid labeling. J of Figure 5 shows the M+0/M+2 citric acid content after ¹³C lactic acid labeling.

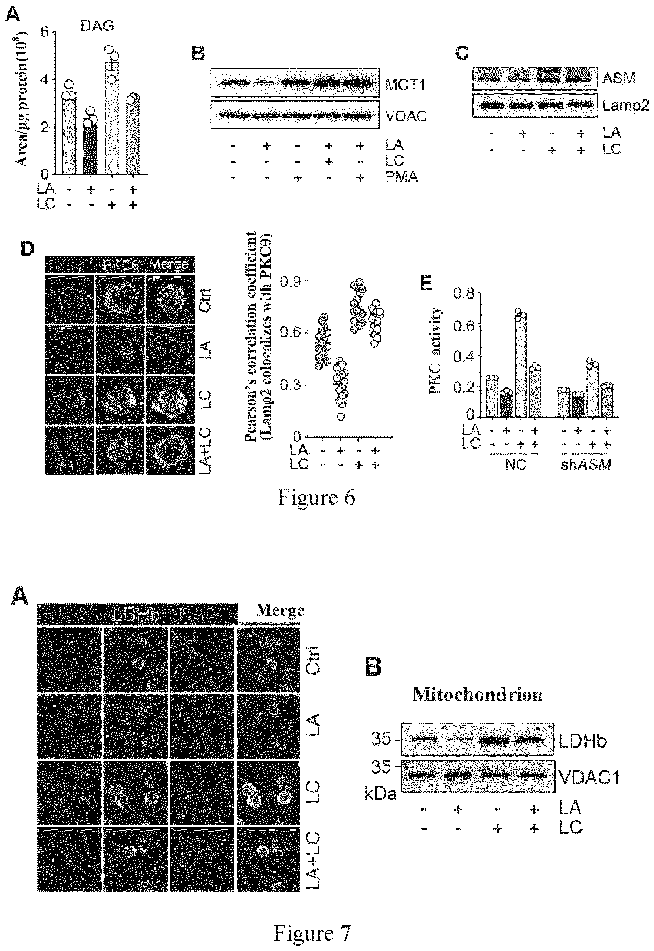

Figure 6 shows that LC activates PKCθ by producing DAG through lysosomal ASM, thereby promoting the translocated expression of MCT1 in mitochondria. A-E of Figure 6 show the changes in intracellular DAG contents after treating with LA and LC for 2 hours (A of Figure 6), mitochondrial MCT1 expression after treating with LA/LC/PMA (B of Figure 6), the changes in lysosomal ASM expression after treating with LC/LC (C of Figure 6), immunofluorescence co-localization of Lamp2 with PKCθ (D of Figure 6), and detection of PKC activity in LA/LC-treated group after silencing ASM (E of Figure 6), respectively.
Figure 7 shows that LC treatment up-regulates mitochondrial LDHb expression. A of Figure 7 shows immunohistochemistry results of LDHb in mitochondria. B of Figure 7 shows the experimental results of Western blotting.

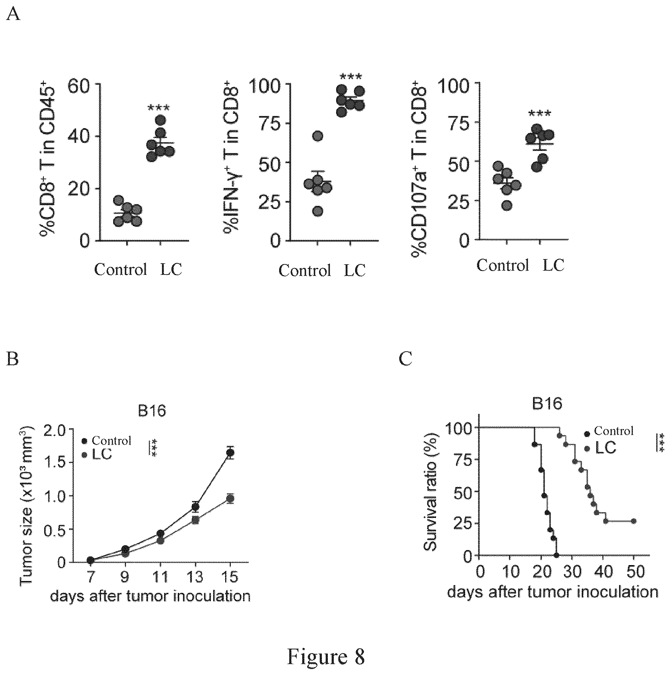

Figure 8 shows that LC treatment can increase the number of tumor-infiltrating CD8⁺ T cells and the expression of the effectors IFN-γ and CD107a in mouse melanoma B16 cell line (A of Figure 8), and can also effectively inhibit the growth of B16 tumors (B of Figure 8) and prolong the survival of mice (C of Figure 8).

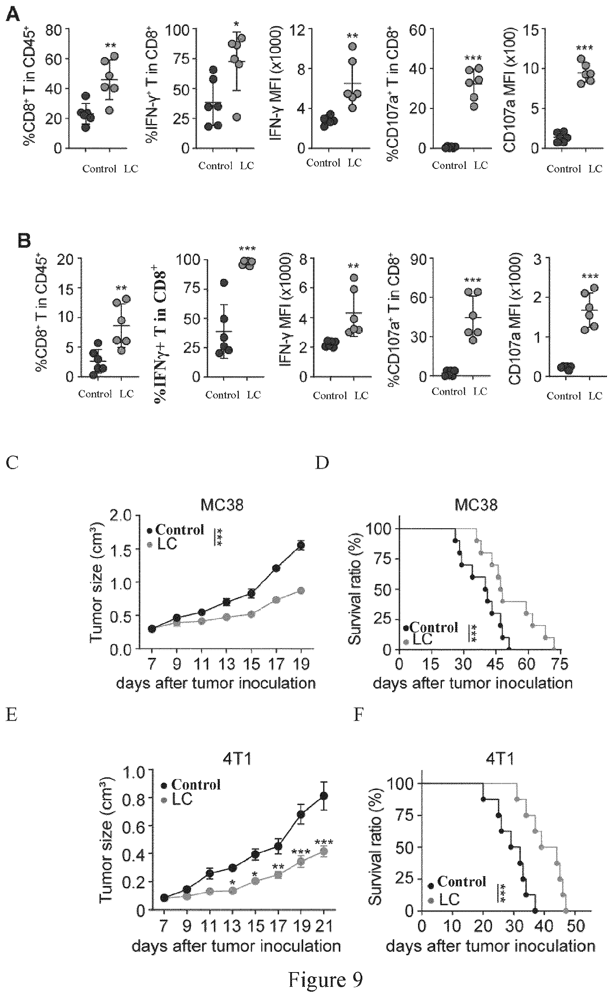

Figure 9 shows that LC treatment can increase the number of tumor-infiltrating CD8⁺ T cells and the expression of the effectors IFN-γ and CD107a in MC38 colon cancer and 4T1 breast cancer tumor-bearing mouse models (A and B of Figure 9), and can also effectively inhibit the growth of MC38 colon cancer (C of Figure 9) and 4T1 breast cancer (E of Figure 9) and prolong the survival of mice (D and F of Figure 9).

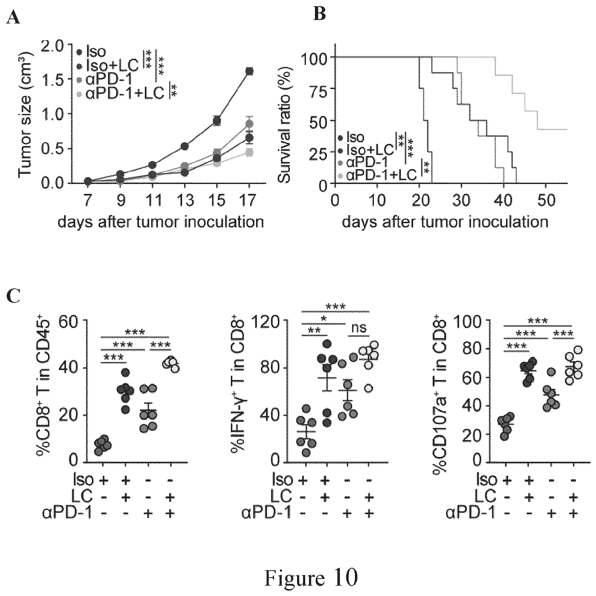

Figure 10 shows that LC can enhance the therapeutic effect of an anti-PD-1 antibody in a B16 tumor-bearing mouse model. A and B of Figure 10 show the tumor size (A of Figure 10) and survival rate of mice (B of Figure 10) after tumor inoculation, respectively. C of Figure 10 shows the number of tumor-infiltrating CD8⁺ T cells and the expression of the effectors IFN-γ and CD107a.

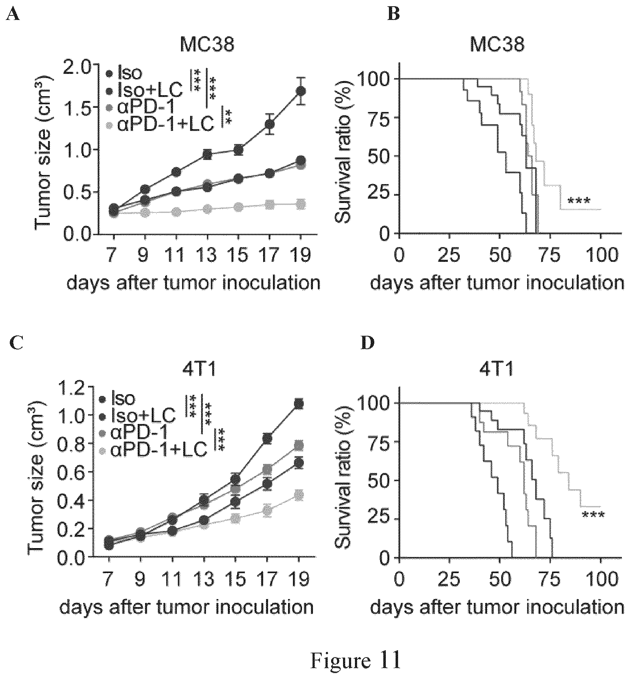

Figure 11 shows that LC can enhance the therapeutic effect of an anti-PD-1 antibody in MC38 as well as 4T1 tumor-bearing mouse models. A and B of Figure 11 show the tumor size (A of Figure 11) and survival rate of mice (B of Figure 11) after tumor inoculation in the MC38 tumor-bearing mouse model, respectively. C and D of Figure 11 show the tumor size (C of Figure 11) and survival rate of mice (D of Figure 11) in the 4T1 tumor-bearing mouse model, respectively.

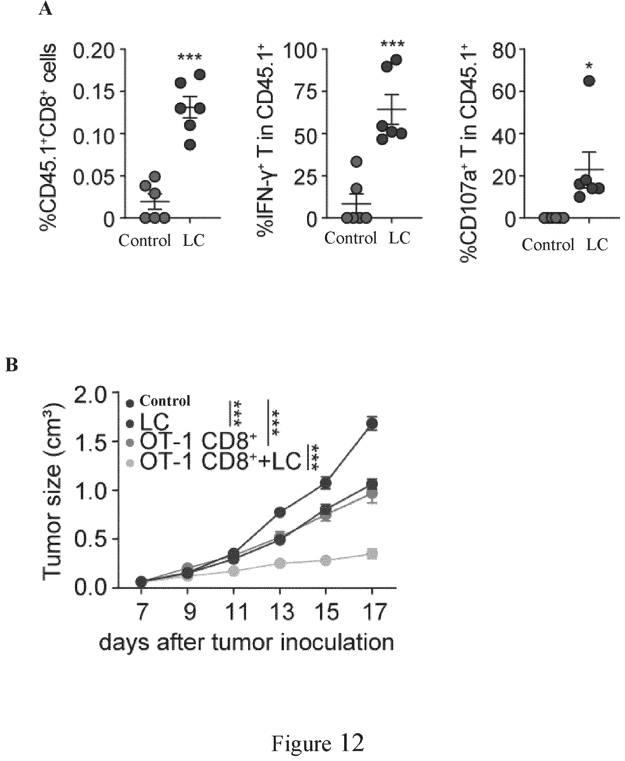

Figure 12 shows that LC treatment enhances the efficacy of T cell adoptive therapy in OVA-B16 melanoma tumor-bearing mice. A of Figure 12 shows that LC treatment can increase the number of tumor-infiltrating CD8⁺ T cells and the expression of the effectors IFN-γ and CD107a. B of Figure 12 shows that LC treatment inhibits tumor growth in mice.

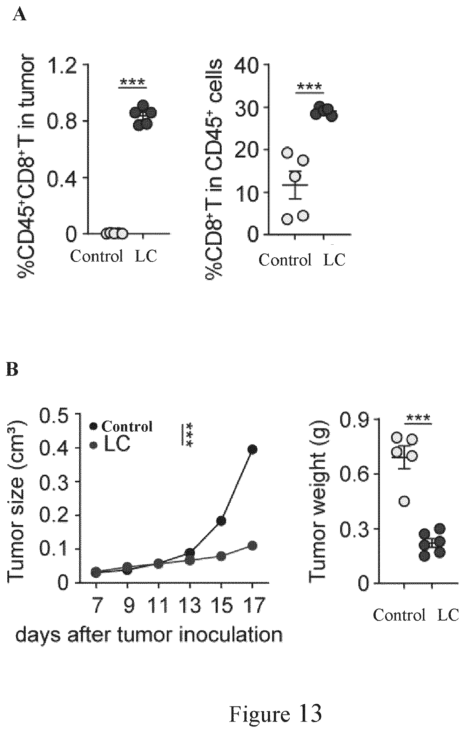

Figure 13 shows the therapeutic effect of LC in a humanized human tumor-bearing mouse model. A of Figure 13 shows that LC treatment significantly increases the infiltration of human-derived CD8⁺ T cells in humanized tumor-bearing mice. B of Figure 13 B shows that LC treatment significantly inhibits tumor growth in mice.

### DETAILED DESCRIPTION OF THE INVENTION

As described herein, the lithium-containing compound of the present invention is selected from the group consisting of lithium carbonate, lithium chloride, lithium hydroxide, n-butyl lithium, lithium sulfate, lithium citrate, lithium orotate, as well as other lithium-containing compounds that can be used or have been used in medicine.

As used herein, the T cell-based tumor immunotherapy includes, but is not limited to, adoptive T cell therapy (ACT) and immune checkpoint inhibitor therapy.

Adoptive T cell therapy is an immunotherapy that utilizes a patient's own immune cells to find and eliminate tumor cells. It utilizes the patient's own (autologous transplant) or donor (allogeneic transplant) immune cells to improve immune function.

Tumor-infiltrating lymphocyte (TIL) adoptive therapy has shown strong efficacy in the treatment of solid tumors, causing objective tumor regression in a variety of cancers such as melanoma, cervical squamous cell carcinoma and cholangiocarcinoma, etc., with about 25% of patients being completely cured and more than half of patients having shrunk metastases.

As immune checkpoint inhibitors, for example, mention may be made of anti-PD-1 antibodies (e.g., nivolumab, pembrolizumab), anti-PD-L1 antibodies (e.g., atezolizumab, avelumab, durvalumab), anti-CTLA-4 antibodies (e.g., ipilimumab and tremelimumab), anti-LAG-3 antibodies (e.g., BMS-986016 and LAG525), etc.

Among them, the PD-1 antibodies can block PD-L1 on tumor cells from binding to the inhibitory receptor PD-1 on the surface of activated T cells, thereby activating tumor-specific T cells and reestablishing the killing effect of the immune system on tumor cells.

### EXAMPLES

The present invention is further illustrated in details with reference to the following examples, and the scope of the present invention is not limited thereto. The experimental methods for which the specified conditions do not indicated in the examples of the present invention generally follow conventional conditions, or the conditions recommended by the raw material or commodity manufacturer. The reagents for which the source is not specified are conventional reagents purchased from the market.

### Example 1. Lactic acid in the tumor microenvironment was transferred to the nucleus of CD8⁺ T cells

It has been demonstrated in literatures (Holm et al., 1995; Walenta and Mueller-Klieser, 2004; Brand et al., 2016; Feder-Mengus et al., 2007; Fischer et al., 2007; Mendler et al., 2012a; Quinn et al., 2020; Zhuang et al., 2010; Uhl et al., 2020) that the tumor microenvironment is an acidic microenvironment dominated by lactic acid, which plays an important role in tumorigenesis and tumor development, and exerts a strong inhibitory effect on anti-tumor T cells. Lactic acid exists in the form of lactate and hydrogen ion, both synergistically enter CD8⁺ T cells through the lactic acid transporter MCT1. By using different mouse tumor models (B16, MC38 and 4T1 subcutaneous tumors), the inventor observed that the lactic acid levels in tumor interstitial fluid gradually increased during the early, middle and late stages of tumor progression, the lactic acid transporter expression and intracellular lactate level of tumor-infiltrating T cells (TILs) were significantly increased (see Figure 1), and the cytoplasm of TILs was significantly acidified as found by flow cytometry using the acid indicator dye pHrodo for staining (results not shown).

Considering that the activity of glycolysis metabolism-associated enzymes depends on the relatively high pH value of cytosol (7.3-7.6), the above results suggested that CD8⁺ T cells were in an inactive state. In order to further explore the effect of lactic acid on CD8⁺ T cells, we used C57BL/6J mouse spleen-derived CD8⁺ T cells to be activated by CD3/CD28 magnetic beads and stimulated by exogenous lactic acid at different concentrations. As detected by flow cytometry, fluorescence quantitative qPCR and ELISA assays, it was found that lactic acid significantly inhibited the activation (decreased CD25 expression, significant down-regulation of the gene and protein expression levels of the effector molecules IL-2, IFN-γ and TNF-α) and proliferation (CTV proliferation assay) of CD8⁺ T cells, significantly promoted the apoptosis of CD8⁺ T cells, and the above effects were in a lactic acid dose-dependent manner (see Figure 2).

CD8⁺ T cells were treated with exogenous hydrochloric acid (HCl) or sodium lactate (NaLa) as controls, and the entry of lactate and hydrogen ions into the cells was detected. It was found that only the lactate and hydrogen ions in the lactic acid-treated group were simultaneously transported into the cells (see Figure 3).

When exogenous lactic acid enters the cell, excess hydrogen ions are further transported to acidic organelles, such as lysosomes, in order to maintain the stability of the cytoplasmic pH values. In our study, exogenous lactic acid was found to rapidly acidify lysosomes, as detected by staining with the lysosomal acidity dye lysosensor. It was found that lactate did not enter lysosomes, as detected by high-performance liquid chromatography-mass spectrometry (LC-MS/MS), and lysosomes did not express MCT1. Meanwhile, we did not detect any distribution of lactate in mitochondria, although lactic acid can be oxidatively metabolized in mitochondria. Surprisingly, we found that m+3 lactic acid was present in the nucleus, but the nucleus did not express MCT1. The use of the nuclear pore blocker wheat germ lectin (WGA) prevented the entry of m+3 lactic acid into the nucleus, suggesting that excess cytoplasmic lactic acid can diffuse into the nucleus through nuclear pores (results not shown).

### Example 2. Histone lactylation in CD8⁺ T cells decreased the anti-tumor activity thereof

We further investigated the effect of lactic acid distributed in the nucleus on the function of CD8⁺ T cells. In 2019, Zhao Yingming's team discovered a new type of histone epigenetic modification, lactylation modification (Kla), which can regulate the phenotype and function of macrophages (Nature. 2019 Oct; 574 (7779):575-580). On this basis, we hypothesized that lactic acid entering the nucleus would lead to lactylation modification of histones, thereby regulating the function of CD8⁺ T cells. After treating CD8⁺ T cells with exogenous lactic acid, the content of lactyl-CoA, a direct donor of histone lactylation, was found to be significantly increased as detected by high-performance liquid chromatography-mass spectrometry (LC-MS/MS), and the level of histone lactylation was found to be significantly increased as detected by Western blotting (anti-pan-Kla antibody), and the above effects were significantly inhibited after pre-treating with a lactic acid transporter MCT1 inhibitor, AZD. In line with this, AZD treatment significantly restored the inhibitory effect on CD8⁺ effector secretion (IL-2/IFN-γ/TNF-α), T cell proliferation, and activation (CD25 expression), induced by exogenous lactic acid (see Figure 4).

In order to further elucidate the mechanism by which histone lactylation modification regulates T cell function, the downstream target genes regulated by histone lactylation modification were explored by using ChIP-Seq experiments, and the enrichment scores of genes corresponding to histone lactylation sites were calculated. KEGG analysis revealed that histone lactylation was not enriched in T cell effector gene sites, but in lysosome-related gene (such as CTSD, TAP1, ATP6V0C, etc.) sites, in particular, ATP6V0C gene sites showed a high degree of histone lactylation. ATP6V0C gene encodes the V0 subunit C protein in V-ATPase that plays a key role in pumping H⁺, which is responsible for pumping cytosolic H⁺ into the lysosomal lumen and thus maintaining the low pH value of lysosomes. It was further found that exogenous lactic acid significantly up-regulated the protein expression of V0C and the activity of V-ATPase in CD8⁺ T cells, which was consistent with the above results of lysosomal pH value decreasing from 5.4 to 4.4. Therefore, exogenous lactic acid results in lactylation of histones and thus up-regulating ATP6V0C expression and activity, thereby shunting cytosolic H⁺ into lysosomes in response to cytosolic acid stress, but lysosomal acidification may inhibit the activity of CD8⁺ T cells.

### Example 3. Lithium carbonate promoted the shunt of exogenous lactic acid to mitochondria of CD8⁺ T cells for oxidative metabolism

The above studies demonstrates that lactic acid (lactate in synergy with H⁺) in the tumor microenvironment is transported into CD8⁺ T cells through MCT1 on the surface of cell membrane of the cells, and the accumulation of lactate ions in the nucleus mediates histone lactylation, modifies lysosome-related genes to promote the up-regulation of V-ATPase expression and activity, and pumps a large amount of H⁺ into lysosomes to cause their acidification, thereby promoting the functional inhibition of CD8⁺ T cells. Although targeting MCT1 is an effective strategy, it affects the uptake and utilization of lactic acid by normal cells such as hepatocytes through MCT1. It is a better strategy to shunt exogenous lactic acid to mitochondria to provide cells with energy via oxidative metabolism through the tricarboxylic acid (TCA) cycle. Studies have shown that lithium carbonate (Li₂CO₃, LC) can significantly down-regulate lactic acid levels in the cerebrospinal fluid of patients with bipolar disorder (*J Clin Psychopharmacol.*2017;37(1):40-45), suggesting that LC may mediate the oxidative metabolism of lactic acid.

It was further found that LC treatment counteracted the elevation in lactic acid levels in activated CD8⁺ T cells caused by exogenous lactic acid, as revealed by ¹³C-lactic acid tracing. LC-mediated decrease in intracellular lactic acid levels may be due to 1) inhibition of uptake of extracellular lactic acid by LC, 2) promotion of efflux of intracellular lactic acid by LC, and 3) promotion of oxidative metabolism of intracellular lactic acid by LC. Experiments demonstrated that LC did not affect the uptake of exogenous lactic acid by CD8⁺ T cells, but reduced the level of extracellular lactic acid. ²D-lactic acid tracing showed that lactic acid was highly accumulated in mitochondria 2 hours after treating CD8⁺ T cells with LC. After 24 hours, mitochondrial m+3 lactic acid, nuclear m+3 lactic acid and lactyl-CoA were found to be almost undetectable as detected by ¹³C-lactic acid tracing. Meanwhile, the levels of intracellular lactic acid and histone Kla were significantly lower than those in the control group, but more oxidation products of lactic acid were detected, including m+2 citric acid and m+4 malic acid, etc. Taken together, these results suggest that LC introduces exogenous lactic acid into mitochondria for oxidative metabolism (see Figure 5).

### Example 4. Lithium carbonate promoted localization of MCT1 to mitochondria by inducing PKCθ activation

The inventor further explored the molecular mechanism by which LC led to the shunting of lactic acid to mitochondria. Entry of lactic acid into mitochondria requires the localization of MCT1 to the inner mitochondrial membrane IMM (Redox Biol. 2020 Aug;35:101454). Western blot analysis of isolated T cell mitochondria showed that the expression of MCT1 in mitochondria in the lactic acid-treated group was significantly reduced, but the expression of MCT1 in mitochondria was significantly increased after LC treatment. MCT1 is generally recognized as a secreted protein with an endoplasmic reticulum-targeting sequence that enters the endoplasmic reticulum and localizes to the plasma membrane. We hypothesized that LC could induce MCT1 variants with mitochondrial targeting sequences (MTSs). However, no MCT1 variant was found in LC-treated T cells by sequencing analysis, and only one transcript of MCT1 was found after alignment analysis on NCBI. A MTS was found at the N-terminus of MCT-1 protein by protein sequence analysis, which was present downstream of the endoplasmic reticulum targeting sequence, forming a double-peak chimeric sequence. This double-peak sequence may direct MCT-1 to target the endoplasmic reticulum or mitochondria, and may be regulated by serine/threonine phosphorylation of PKA or PKC (Avadhani, N.G. 2011. FEBS J 278, 4218-4229). Further studies found that the use of PKA, PKCβ or PKC (α, γ, η) inhibitors did not block the localization of MCT1 to mitochondria induced by LC, whereas the PKCθ inhibitor Sotrastaurin almost completely blocked the localization of MCT1 to mitochondria induced by LC (results not shown).

PKCθ is a new member of the PKC family, which is activated by DAG and is widely expressed in T lymphocytes. We found that LC treatment increased DAG levels in CD8⁺ T cells, and the use of the DAG analog phorbol ester (PMA) significantly increased the localization of MCT1 to mitochondria. Further studies found that the expression of acid sphingomyelinase (ASM) in lysosomes was increased. This enzyme has PLC activity and catalyzes the production of DAG from lysosomal phospholipids. Meanwhile, it was found that LC treatment could induce the localization of PKCθ to lysosomes. shASM was used to interfere with the expression of ASM, and it was found that LC treatment could not induce PKCθ activation. These results suggest that LC activates PKCθ by producing DAG through lysosomal ASM, thereby promoting the translocation of MCT1 to mitochondria (see Figure 6).

### Example 5. LC-triggered activation of TFEB induced up-regulated expression of LDHb

The above results indicate that the activity of lysosomal PLC is enhanced after LC treatment, and PKCθ is activated by DAG, thereby regulating the localization of MCT1 to mitochondria. The lactic acid that enters the mitochondria is further converted into pyruvic acid with the participation of lactate dehydrogenase to enter the TCA cycle for oxidative metabolism. *LDHa* and *LDHb* are two major lactate dehydrogenase genes that encode LDHa (also known as M subunit) and LDHb (H subunit). The five LDH isozymes from LDH1 to LDH5 consist of M and H subunits, and are 4H, 3H1M, 2H2M, 1H3M and 4M, respectively. In this study, it was found that LC treatment up-regulated the expression of LDHa and LDHb. LDHa is mainly expressed in the cytosol, whereas LDHb is expressed only in the inner mitochondrial membrane. Considering that LDHa has a higher affinity for pyruvic acid, LDHb has a higher affinity for lactic acid (Proteins. 2001 May 1;43(2):175-85), therefore, lactic acid that entered the mitochondria was converted to pyruvic acid under the catalysis of the isoenzyme LDH1 (see Figure 7).

Furthermore, this study analyzed the molecular mechanism bu which LC promotes the localization and expression of LDHb in mitochondria. The above results show that both LA and LC play a role by targeting lysosomes. TFEB is a major regulator of lysosomal functions. We found that the expression and nuclear localization of TFEB were significantly up-regulated after LC treatment, indicating that TFEB was activated by dephosphorylation and was translocated to the nucleus to exert its function as a transcription factor. siRNA was used to silence the expression of TFEB, and it was found that the up-regulation of LDHb expression caused by LC was significantly inhibited. It was found that TFEB did bind to the promoter region of LDHb gene in LC-treated CD8⁺ effector T cells, as detected by ChIP-qPCR analysis, and furthermore, TFEB was verified to regulate the expression of LDHb by luciferase reporter analysis. In addition, Tfeb^{fl/fl} Lck^{cre} mice were constructed in this study, and CD8⁺ T cells were isolated from the spleen of mice to repeat the above experiments. It was found that ¹³C-labeled lactic acid could not be oxidatively metabolized in the TCA cycle. In conclusion, the above results suggest that LC up-regulates LDHb expression through TFEB signaling, and converts the lactic acid transported by MCT1 to pyruvic acid in mitochondria (results not shown).

### Example 6. LC promoted Ca2+ release in lysosomes by increasing lysosomal pH value

Next, we investigated the molecular mechanism by which LC activated TFEB in CD8⁺ T cells. It has been reported (Nat Cell Biol. 2015 Mar;17(3):288-99) that in addition to being able to degrade exogenous or endogenous biomacromolecules, lysosomes, like endoplasmic reticulum and mitochondria, are also storage sites for Ca²⁺ and can initiate the transduction of calcium-related downstream signals. We found that cytosolic Ca²⁺ concentrations were increased after LC treatment, and blocking the calcium release from endoplasmic reticulum or mitochondria with Ryanodine or CGP37157 did not affect the increase in cytosolic Ca²⁺ concentrations in CD8⁺ T cells induced by LC. These results suggest that lysosomes may act as acidic organelles and store and release Ca²⁺ through Ca²⁺ channels (TPC1/2 and Mcoln1/2). In this study, it was found that LC-treated CD8⁺ T cells had increased expression of Mcoln2. siRNA was used to silence the expression of Mcoln2, and it was found that the Ca²⁺ release, TFEB nuclear localization, and expression of LDHb induced by LC were significantly inhibited (results not shown).

A series of studies have found that elevated lysosomal pH value leads to Ca²⁺ release (NC, CMI, J Cell Sci. 2002;115(Pt 3):599-607). In this study, it was also found that increasing lysosomal pH value with bafilomycin A1 or chloroquine (CQ) would also promote Ca²⁺ release in lactic acid-treated CD8⁺ T cells. LA treatment resulted in a decrease in lysosomal pH, but we found that LC treatment counteracted the effects of LA and increased the lysosomal pH from 4.4 to 5.9 within 30 minutes. The lysosome-related gene Atp6v0c was significantly up-regulated in the exogenous lactic acid-treated group, but was significantly decreased after LC treatment. Further analysis of ChIP-seq data demonstrated that the Atp6v0c gene had a high level of lactylation in the lactic acid-treated group, but the lactylation disappeared after LC treatment. In order to better track the flow of H⁺, ²D (deuterium)-acetic acid (DAC) or ²D-sulfuric acid (D₂SO₄) was added to the culture medium of CD8⁺ T cells. It was found that LC treatment significantly inhibited the entry of deuterium ions into the lysosomal lumen, which led us to speculate that LC may interfere with the function of lysosomal V-ATPase to pump H⁺. The K-loop of V-ATPase is located in the hydrophobic lipid bilayer and is essential for the pumping of cytosolic H⁺ into the lysosomal lumen. Li⁺ has been reported to bind to the K-loop of V-ATPase in *Enterococcus hirae* and induce deprotonation of the Glu139 residue (J. Am. Chem. Soc. 2011.133. 2860-2863). In this study, after treating CD8⁺ T cells with LC and extracting the lysosomes for detection, it was also found that Li⁺ was accumulated in lysosomes. Treatment with bafilomycin A1 blocked the entry of Li⁺ into lysosomes, while Ca²⁺ release was reduced. Taken together, these results suggest that LC can pump Li⁺ instead of proton into the lysosomal lumen, resulting in an increase in lysosomal pH value and Ca²⁺ release (results not shown).

### Example 7. LC treatment improved T cell-based tumor immunotherapy

The above results suggest that LC regulates Ca²⁺ release from lysosomes, thereby promoting the oxidative metabolism of lactic acid in the mitochondria of CD8⁺ T cells, which provides a potential strategy for T cell-based tumor immunotherapy. On this basis, we established an LC treatment model of tumor-bearing mice to demonstrate the tumor treatment effect of LC, and the detailed protocol is as follows.

Lithium carbonate is an inorganic compound with the chemical formula Li₂CO₃ and a molecular weight of 73.89. Lithium carbonate was prepared as follows:
1) 1.000 g of lithium carbonate (Sigma, WXBC5202V) was weighed and placed in a beaker containing 50 mL of double-distilled water, and the volume was brought to 100 mL with double-distilled water.
2) The mixture was mixed well in an ice water bath for 2 hours to fully dissolve the lithium carbonate.
3) The solution was aliquoted into 50 mL centrifuge tubes and stored at 4°C.

**Model establishment:** Cultured mouse melanoma B16 cells were resuspended in PBS buffer and inoculated subcutaneously at 1×10⁵ cells/mouse into the right back of the mouse, with an injection volume of 50 µL. The needle of a 1 mL syringe was inserted from the right thigh of the mouse, penetrating under the skin to the back, to inject 50 µL of tumor cell suspension subcutaneously to the mouse to form a translucent lump of skin. Care should be taken not to puncture the skin of the mouse. The needle was quickly withdrawn after the injection, so as to avoid any leaking of tumor cells when withdrawing the needle, which would result in tumorigenesis of multiple subcutaneous tumors. Tumor cells were inoculated as soon as possible to maintain cell viability. After inoculation, the tumor growth in mice was observed every day, and tumorigenesis generally occurred in about seven days. After tumorigenesis, tumor-bearing mice with uniform body weight and subcutaneous tumor size were selected and randomly divided into two groups:
1) Control group: Starting from Day 7, tumor-bearing mice were given the same dose of solvent (sterile water, 10 µL/g mouse) by gavage once every two days for a total of 5 doses.
2) LC group: Starting from Day 7, tumor-bearing mice were given lithium carbonate (75 mg/kg, i.g. q.d.) by gavage administration once every two days for a total of 5 doses.

During the administration of lithium carbonate, the blood lithium concentration of mice was dynamically monitored and maintained at 1 mM. The volume of subcutaneous tumors in mice was measured every other day and the survival time of mice was observed. The proportion and function of tumor-infiltrating CD8⁺ T cells were analyzed on Day 18. It was found that LC treatment could increase the number of tumor-infiltrating CD8⁺ T cells and the expression of the effectors IFN-γ and CD107a, and could also effectively inhibit the growth of B16 tumors and prolong the survival of mice (see Figure 8).

The present inventor further established MC38 colon cancer and 4T1 breast cancer tumor-bearing mouse models. The tumor-bearing mice were treated in the same manner as the B16 model described above, and similar results were obtained (see Figure 9).

### Example 8. Combination with anti-PD-1 antibody enhanced the anti-tumor treatment effect of LC

Anti-PD-1 antibodies represent another important T cell-based immunotherapy. Most patients exhibit significant therapeutic effect at the beginning of treatment, but still have relapse despite the presence of T cells in the tumor tissue, which means that other factors in the tumor microenvironment may be influencing the effect of the PD-1 antibody. Recent studies have shown that lactic acid plays an important role in modulating the resistance to PD-1 antibody, prompting us to examine whether LC can improve the effect of PD-1 antibody blockade therapy.

After tumorigenesis, the mice were divided into four groups:
1) Control group: Starting from Day 7, tumor-bearing mice were given the same dose of solvent (sterile water, 10 µL/g mouse) by gavage once every two days for a total of 5 doses.
2) LC group: Starting from Day 7, tumor-bearing mice were given lithium carbonate (75 mg/kg, i.g. q.d.) by gavage administration once every two days for a total of 5 doses.
3) Anti-PD-1 antibody group: Starting from Day 7, tumor-bearing mice were given intraperitoneal injection of anti-PD-1 antibody (biotin anti-mouse CD279, Biolegend) at 100 µg/mouse/dose once every two days for a total of 5 doses.
4) LC and anti-PD-1 antibody combined therapy group: On the basis of the administration of Group 2, starting from Day 7, tumor-bearing mice were given intraperitoneal injection of anti-PD-1 antibody (biotin anti-mouse CD279, Biolegend) at 100 µg/mouse/dose once every two days for a total of 5 doses.

During the administration of lithium carbonate, the blood lithium concentration of mice was dynamically monitored and maintained at 1 mM. After 7 days of tumorigenesis, the volume of subcutaneous tumors in mice was measured every other day and the survival time of mice was observed. The proportion and function of tumor-infiltrating CD8⁺ T cells were analyzed on Day 18. It was found that LC and anti-PD-1 antibody combined therapy could increase the number of tumor-infiltrating CD8⁺ T cells and the expression of the effectors IFN-γ and CD107a, and could effectively inhibit the growth of B16 tumors and prolong the survival of mice, compared with the groups treated with a single agent (see Figure 10).

The present inventors further established MC38 colon cancer and 4T1 breast cancer tumor-bearing mouse models. The tumor-bearing mice were treated in the same manner as the B16 model described above, and similar results were obtained (see Figure 11).

### Example 9. T-cell adoptive therapy

In order to further verify the anti-tumor immune response specific for CD8⁺ T cells induced by LC, on the basis of the established tumor-bearing mice above (Example 7), 1×10⁶ CD45.1 OT-1 T tumor-specific CD8⁺ T cells were sorted and adoptively transferred through the tail vein to wild-type C57 mice 24 hours before the subcutaneous tumor inoculation. After 24 hours, 1×10⁵ OVA-B16 melanoma cells were subcutaneously inoculated into the right back of the mice. After 7 days of tumorigenesis, the mice were divided into four groups as follows.
1) Control group: PBS was injected through the tail vein on Day -1. Starting from Day 7, tumor-bearing mice were given the same dose of solvent (sterile water, 10 µL/g mouse) by gavage once every two days for a total of 5 doses.
2) LC-treated group: PBS was injected through the tail vein on Day -1. Starting from Day 7, tumor-bearing mice were given lithium carbonate (75 mg/kg, i.g. q.d.) by gavage administration once every two days for a total of 5 doses.
3) Adoptive therapy group: 1×10⁶ CD45.1 OT-1 T tumor-specific CD8⁺ T cells were adopted through the tail vein on Day -1. Starting from Day 7, tumor-bearing mice were given the same dose of solvent (sterile water, 10 µL/g mouse) by gavage once every two days for a total of 5 doses.
4) Adoptive therapy + LC-treated group: 1×10⁶ CD45.1 OT-1 T tumor-specific CD8⁺ T cells were adopted through the tail vein on Day -1. Starting from Day 7, tumor-bearing mice were given lithium carbonate (75 mg/kg, i.g. q.d.) by gavage administration once every two days for a total of 5 doses.

During the administration of lithium carbonate, the blood lithium concentration of mice was dynamically monitored and maintained at 1 mM. After 7 days of tumorigenesis, the volume of subcutaneous tumors in mice was measured every other day and the survival time of mice was observed. The proportion and function of tumor-infiltrating CD8⁺ T cells were analyzed on Day 18. It was found that LC treatment could increase the number of tumor-infiltrating CD8⁺ T cells and the expression of the effectors IFN-γ and CD107a, and could inhibit the tumor growth in mice (see Figure 12).

### Example 10. LC treatment regimen for humanized tumor-bearing mice

The above results indicate that LC has a good therapeutic effect in multiple tumor-bearing mouse models, and can exert better anti-tumor effect when in combination with PD-1 neutralizing antibodies. Accordingly, it is necessary to further verify the therapeutic effect of the drug in human tumors. Therefore, we used a humanized human tumor-bearing mouse model to investigate the therapeutic effect of LC.

Model establishment: NOD.Cg-Prkdc<scid>Il2rg<tm1Wjl>SzJ (NSG) mice were irradiated with X-ray irradiation (2 Gy). 10⁷ PBMCs from healthy volunteers were adopted into NSG mice through the tail vein within 24 hours. After 24 hours, human A375 tumor cells were digested and counted, 4×10⁵ of which were inoculated subcutaneously into the right back of NSG mice, and tumorigenesis generally occurred in about seven days. After tumorigenesis, tumor-bearing mice with uniform body weight and subcutaneous tumor size were selected and randomly divided into two groups.
1) Control group: Starting from Day 7, tumor-bearing mice were given the same dose of solvent (sterile water, 10 µL/g mouse) by gavage once every two days for a total of 5 doses.
2) LC-treated group: Starting from Day 7, tumor-bearing mice were given lithium carbonate (75 mg/kg, i.g. q.d.) by gavage administration once every two days for a total of 5 doses.

During this period, the blood lithium concentration of mice was dynamically monitored and maintained at 1 mM. The volume of subcutaneous tumors in mice was measured every other day. The proportion of tumor-infiltrating human CD8⁺ T cells was analyzed on Day 18. It was found that LC treatment significantly increased the infiltration of human CD8⁺ T cells in humanized tumor-bearing mice and inhibited the tumor growth in mice (see Figure 13).

## Claims

1. Use of a lithium-containing compound in preparing a medicament for treating a tumor.

2. Use of a lithium-containing compound in preparing a medicament for enhancing immune checkpoint inhibitor and/or adoptive T cell therapy.

3. Use of a lithium-containing compound in combination with an immune checkpoint inhibitor in preparing a medicament for treating a tumor.

4. The use according to any one of claims 1 to 3, wherein the lithium-containing compound is selected from one or more of lithium carbonate, lithium chloride, lithium hydroxide, n-butyl lithium, lithium sulfate, lithium citrate and lithium orotate.

5. The use according to claim 2 or 3, wherein the immune checkpoint inhibitor is selected from the group consisting of an anti-PD-1 antibody, an anti-PD-L1 antibody, an anti-CTLA-4 antibody and an anti-LAG-3 antibody.

6. The use according to any one of claims 1 to 3, wherein the tumor is selected from the group consisting of head and neck cancer, esophageal cancer, gastric cancer, colon cancer, rectal cancer, liver cancer, gallbladder cancer, cholangiocarcinoma, pancreatic cancer, lung cancer, breast cancer, ovarian cancer, bladder cancer, prostate cancer, testicular cancer, bone and soft tissue sarcoma, malignant lymphoma, leukemia, cervical cancer, skin cancer, brain tumor and melanoma.

7. The use according to any one of claims 1 to 3, wherein the lithium-containing compound exerts anti-tumor activity by promoting the utilization of lactic acid in the microenvironment by tumor-specific CD8⁺ T cells, preferably, the lithium-containing compound is formulated into a dosage form for oral, intradermal, subcutaneous, intraperitoneal or intravenous administration.

8. A pharmaceutical composition containing a lithium-containing compound and an immune checkpoint inhibitor, as well as one or more pharmaceutically acceptable carriers or excipients.

9. The pharmaceutical composition according to claim 8, wherein the lithium-containing compound is selected from one or more of lithium carbonate, lithium chloride, lithium hydroxide, n-butyl lithium, lithium sulfate, lithium citrate and lithium orotate.

10. A method for enhancing the anti-tumor activity of a tumor-specific T cell *in vitro,* including the following steps:
1) obtaining a tumor-specific T cell; and
2) contacting the tumor-specific T cell with a lithium-containing compound.
